Europäisches Patentamt

European Patent Office

Office européen des brevets

(11) Publication number: **0 332 234 B1**

## EUROPEAN PATENT SPECIFICATION

(45) Date of publication of patent specification: **28.07.93** (51) Int. Cl.5: **C12N 15/31**, C12P 13/22

(21) Application number: **89108172.1**

(22) Date of filing: **16.02.84**

(60) Publication number of the earlier application in accordance with Art.76 EPC: **0 136 359**

(54) **Process for preparing L-tyrosine.**

(30) Priority: **17.02.83 JP 25397/83**
**17.02.83 JP 25398/83**
**28.05.83 JP 94392/83**
**29.07.83 JP 138775/83**
**04.08.83 JP 142804/83**
**24.09.83 JP 176758/83**

(43) Date of publication of application:
**13.09.89 Bulletin 89/37**

(45) Publication of the grant of the patent:
**28.07.93 Bulletin 93/30**

(84) Designated Contracting States:
**AT BE CH DE FR GB LI NL SE**

(56) References cited:
**EP-A- 0 066 129**

(73) Proprietor: **KYOWA HAKKO KOGYO CO., LTD.**
**6-1, Ohtemachi 1-chome**
**Chiyoda-ku Tokyo 100(JP)**

(72) Inventor: **Oka, Tetsuo**
**2360-17, Naramachi Midori-ku**
**Yokohama-shi Kanagawa 227(JP)**
Inventor: **Katsumata, Ryoichi**
**Popuragaoka Coopo 6-401 12-3, Naruse**
**2-chome**
**Machida-shi Tokyo 194(JP)**
Inventor: **Ozaki, Akio**
**6-6, Asahimachi 3-chome**
**Machida-shi Tokyo 194(JP)**
Inventor: **Mizukami, Toru**
**14-10, Asahimachi 2-chome**
**Machida-shi Tokyo 194(JP)**
Inventor: **Yokoi, Haruhiko**
**6-6, Asahimachi 3-chome**
**Machida-shi Tokyo 194(JP)**
Inventor: **Hara, Masako**
**40-11, Sagamigaoka 5-chome**
**Zama-shi Kanagawa 228(JP)**

(74) Representative: **Vossius & Partner**
**Siebertstrasse 4 P.O. Box 86 07 67**
**W-8000 München 86 (DE)**

EP 0 332 234 B1

**Description**

This invention relates to a process for producing L-tyrosine by a novel method for the expression of a gene. More specifically, the present invention relates to a process for producing L-tyrosine by transforming a host microorganism belonging to the genus Corynebacterium or Brevibacterium with a recombinant DNA of a DNA fragment containing a gene coding for 3-deoxy-2-keto-D-arabino-heptulosonate 7-phosphate synthetase (referred to as DAHPase hereinafter), chorismate mutase (referred to as CMase hereinafter) and prephenate dehydrogenase (referred to as PDGase hereinafter) and a vector DNA, culturing the transformant in a nutrient medium, accumulating L-tyrosine in the culture medium and recovering it therefrom.

For the direct production of amino acids by fermentation, methods using wild-type strains, or mutant strains such as auxotrophic strains and amino acid analog-resistant strains, of the bacteria belonging to the genus Corynebacterium, Brevibacterium, Serratia, and the like are known.

For example, the production of histidine using a strain resistant to histidine analog (Japanese Patent Publication No. 8596/81), production of tryptophan using a strain resistant to tryptophan analog (Japanese Patent Publication Nos. 4505/72 and 19037/76), production of isoleucine using a strain resistant to isoleucine analog (Japanese Patent Publication Nos. 38995/72, 6237/76 and 32070/79), production of phenylalanine using a strain resistant to phenylalanine analog (Japanese Patent Publication No. 10035/81), production of tyrosine using a strain requiring phenylalanine for its growth or being resistant to tyrosine (Agr. Chem. Soc., Japan 50 (1) R79-R87 (1976)], production of arginine using a strain resistant to L-arginine analog [Agr. Biol. Chem., 36, 1675-1684 (1972), Japanese Patent Publication Nos. 37235/79 and 150381/82] and the like are known.

The present invention relates to the production of L-tyrosine using a microorganism belonging to the genus Corynebacterium or Brevibacterium by recombinant DNA technology different from the conventional mutational breeding technology for the purpose of improving the L-tyrosine productivity.

The present inventors have constructed plasmid vectors autonomously replicable in a microorganism belonging to the genus Corynebacterium or Brevibacterium and having selectable markers and adequate cloning sites and have developed a highly efficient transformation system (Japanese Published Unexamined Patent Application Nos. 183799/82, 186492/82 and 186489/82). Further, the present inventors have found that the plasmid vectors are useful for expressing a foreign gene in a host microorganism and increasing the productivity of amino acids by ligating a DNA fragment containing a foreign gene involved in the biosynthesis of amino acids such as glutamic acid and lysine to the plasmid vectors according to the procedures in recombinant DNA technology (Methods in Enzymology 68, Recombinant DNA, edited by Ray Wu, Academic Press 1979, U.S. Patent No. 4,237,224) and transforming Corynebacterium glutamicum L-22 or its derivatives using the transformation methods developed by the present inventors (Japanese Published Unexamined Patent Application No. 126789/83).

The present inventors have found that microorganisms prepared by the above method acquire an increased productivity of tyrosine.

No example of expressing a desired gene and increasing the productivity of L-tyrosine in a host microorganism belonging to the genus Corynebacterium or Brevibacterium by introducing a recombinant DNA containing such desired gene and vector, one of which is foreign to a host microorganism, into such host microorganism has been reported.

The present invention is explained in detail below.

The present invention provides a process for producing L-tyrosine by cultivating in a medium a transformant which is obtained by transforming a microorganism belonging to the genus Corynebacterium or Brevibacterium with a recombinant DNA of a DNA fragment containing a gene coding for DAHPase, CMase and PDGase and a vector DNA.

As the DNA fragment containing the gene used in the present invention, the DNA fragment containing a gene coding for DAHPase, CMase and PDGase of the present invention derived from eukaryotes, prokaryotes, viruses, bacteriophages or plasmids is used. As the gene derived from prokaryotes, the gene derived from a bacterium belonging to the genus Escherichia, Corynebacterium, Brevibacterium, Microbacterium, Bacillus, Staphylococcus, Streptococcus or Serratia and coding for DAHPase, CMase and PDGase or the metabolism relating to the biosynthesis is preferably used.

As the most preferable source of the gene, amino acid-producing strains and amino acid analog-resistant strains derived from the bacteria described above are used. The amino acid analog-resistance can be conferred on a plasmid after cloning.

As the source of the gene coding for DAHPase, CMase and PDGase of the present invention, Escherichia coli JA194 [Proc. Natl. Acad. Sci., 74, 487-491 (1977)] is preferably used.

2

Metabolic pathway and regulation systems of aromatic amino acids in microorganisms have been studied in detail on Escherichia coli, Bacillus subtilis, and glutamic acid-producing microorganisms such as strains of the genus of Corynebacterium and Brevibacterium [Agr. Chem. Soc. Japan, 50 (1), R79 - R87 (1976) and Ann. Rev. Biochemistry 47, 533 (1978)]. The gene coding for DAHPase, CMase and PDGase of the present invention is a DNA carrying a genetic information of at least one of enzymes involved directly or indirectly in the biosynthesis of these aromatic amino acids. The genes encoding for enzymes subjected to regulation on the biosynthetic pathway, i.e. DAHPase CMase PDGase hereinafter) and pretyrosine aminotransferase are preferably used. Further, the genes of strains which are relieved from feed back inhibition and repression a priori or a posteriori are applicable.

The vector used in the present invention should autonomously replicate in cells of the host microorganism. Preferably, plasmids isolated from microorganisms belonging to the genus Corynebacterium by the present inventors or derivatives thereof such as pCG1 (Japanese Published Unexamined Patent Application No. 134500/82), pCG2 (Japanese Published Unexamined Patent Application No. 35197/83), pCG4 (Japanese Published Unexamined Patent Application No. 183799/82), pCE51, pCE52 (Japanese Published Unexamined Patent Application No. 126789/83), pCE53 (Japanese Published Unexamined Patent Application No. 25398/83, pCE54, pCG11, pCB100 (Japanese Published Unexamined Patent Application No. 105999/83), and the like are mentioned.

Microorganisms carrying these plasmids have been deposited with the Fermentation Research Institute, Agency of Industrial Science and Technology, and the American Type Culture Collection, U.S.A. under the following accession numbers.

| Plasmid | FERM P- | ATCC |
|---------|---------|-------|
| pCG1 | 5865 | 31808 |
| pCG2 | 5954 | 31832 |
| pCG4 | 5939 | 31830 |
| pCE54 | - | 39019 |
| pCG11 | - | 39022 |
| pCB101 | - | 39020 |

Plasmids pCE51, 52, 53 and 54 can be introduced from the plasmids mentioned above as follows.

For example, pCE51 is prepared as follows.

pCG1 is isolated from the cultured cells of Corynebacterium glutamicum 225-57 (FERM P-5865, ATCC 31808) by the method described in the specification of Japanese Published Unexamined Patent Application No. 134500/82. pGA22 is isolated from the cultured cells of Escherichia coli harboring the plasmid by a conventional method [An, G. et al.,: J. Bacteriol., 140, 400 (1979)]. Plasmid pCG1 is linearized with restriction endonuclease BglII and a fragment of pGA22 digested with BamHI and containing kanamycin resistance (Km$^R$) gene is ligated to the linearized pCG1 using the same cohesive ends of both plasmids. Isolation of pCE51 from the ligated DNA mixture is achieved by selecting the transformants belonging to the genus Corynebacterium or Brevibacterium and containing Km$^R$ derived from pGA22, and analyzing the plasmid in the transformant.

pCE51 has a molecular weight of about 6 Kb and cleavage sites for HincII, HindIII, SmaI, XhoI and EcoRI and gives Km$^R$ phenotype.

pCE52 and pCE53 are prepared as follows.

Plasmid pCG1 is isolated from the cultured cells of Corynebacterium glutamicum 225-57 (FERM P-5865, ATCC 31808) by the method described in the above application and plasmid pGA22 is isolated from the cultured cells of Escherichia coli harboring the plasmid by a conventional method. pCG1 with a unique BglII site is linearized with restriction enzyme BglII and pGA22 with two BamHI sites are partially digested with BamHI. The cohesive ends of both plasmids are annealed and ligated with T4 phage DNA ligase to make a composite molecule. Selection of the recombinant plasmids from the ligation mixture is carried out by isolating transformants of the genus Corynebacterium or Brevibacterium on the basis of drug-resistances derived from pGA22 and then analyzing the plasmids in the transformants.

pCE52 and pCE53 have a molecular weight of about 10.9 Kb and cleavage sites for EcoRI, SalI, SmaI and XhoI. While pCE52 gives the phenotypes of resistance to chloramphenicol (Cm$^R$) and Km$^R$, pCE53 gives resistance to tetracycline (Tc$^R$), Cm$^R$ and Km$^R$ phenotypes. Since the cleavage site for XhoI is present in the Km$^R$ gene, selection by insertional inactivation (prevention of the expression of a gene by the insertion of a DNA fragment into the gene) is possible.

Transformation with the ligated DNA mixture is carried out using protoplasts of the genus Corynebacterium or Brevibacterium, and the method described in Japanese Published Unexamined Patent Application Nos. 186492/82 and 186489/82.

Among the genes responsible for drug resistance derived from pGA22, those except for the resistance gene which is insertionally inactivated are used for selection. In no DNA adding system, transformants are recovered as a colony regenerated on a hypertonic agar medium containing generally 0.4 - 1.6 $\mu$g/m$\ell$ Tc, 2.5 - 5 $\mu$g/m$\ell$ Cm, 100 - 800 $\mu$g/m$\ell$ Km, 100 - 400 $\mu$g/m$\ell$ Sm or 200 - 1,000 $\mu$g/m$\ell$ Spec which does not allow the reversion to normal cells of the recipient protoplasts which are not treated with the ligation mixture. Alternatively, transformants are regenerated unselectively on a regeneration medium, and the resultant cells are scraped and resuspended, followed by the isolation of those cells grown on an agar medium containing a drug in a concentration wherein the recipient normal cells can not grow, that is, generally 0.5 - 4 $\mu$g/m$\ell$ Tc, 2 -15 $\mu$g/m$\ell$ Cm, 2 -25 $\mu$g/m$\ell$ Km, 5 - 50 $\mu$g/m$\ell$ Sm or 50 -500 $\mu$g/m$\ell$ Spec. Some of the transformants selected by $Tc^R$, $Cm^R$ or $Km^R$ are simultaneously endowed with other drug-resistances derived from plasmid pGA22.

Plasmid DNAs in these transformants can be isolated from cultured cells of the transformants and purified according to the methods described in Japanese Published Unexamined Patent Application Nos. 134500/82 and 186489/82. The structures of the DNAs can be determined by digesting them with various restriction endonucleases and analyzing the DNA fragments by agarose gel electrophoresis. The plasmids isolated from the transformants are pCE51, pCE52 and pCE53. Recovery of plasmids from the strains is carried out according to the methods described in Japanese Published Unexamined Patent Application Nos. 134500/82, 183799/82 and 35197/83. Preparation of a recombinant DNA of a vector DNA with a DNA fragment containing a gene is carried out by conventional in vitro recombinant DNA technology.

In vitro recombinant DNA technology is carried out by cleavage and ligation of a donor DNA containing a desired gene to a vector DNA (refer to Japanese Published Unexamined Patent Application No. 126789/83, USP 4,237,224).

The ligase reaction gives recombinants containing genes other than the desired gene. The desired recombinant DNA can be obtained by directly transforming a microorganism of the genus Corynebacterium or Brevibacterium with the ligated DNA mixture, selecting the transformants having the phenotype derived from the desired gene and isolating the desired recombinant DNA from the cultured cells of the transformants. Instead of cloning the desired gene directly in a microorganism of the genus Corynebacterium or Brevibacterium, the desired gene can be cloned by using another host-vector system such as Escherichia coli. Then, it is recloned in vitro into a vector of the genus Corynebacterium or Brevibacterium to transform these microorganisms and transformants containing the desired recombinant plasmid are selected as mentioned above.

The method of cloning a gene in a host microorganism of Escherichia coli is described in, for example, Methods in Enzymology, 68, Ray Wu (Ed) Academic Press New York (1979).

The following references are helpful for the construction of recombinant DNA:

S.N. Cohen, et al., U.S.P. No. 4,237,224;

Idenshi Sosa Jikkenho, edited by Yasuyuki Takagi, printed by Kodansha Scientific (1980);

Methods in Ezymology 68, Recombinant DNA, edited by Ray Wu, Academic Press, 1979

Japanese Published Unexamined Patent Application No. 126789/83.

Microorganisms belonging to the genus Corynebacterium or Brevibacterium and which are competent for incorporating DNAs may be used as the host microorganisms in the present invention. The following are examples of a suitable host microorganism.

|  |  | Accession Number | |
| --- | --- | --- | --- |
|  |  | FERM P- | ATCC |
| Corynebacterium glutamicum |  |  | 13032 |
| Corynebacterium glutamicum L-15 |  | 5946 | 31834 |
| Corynebacterium glutamicum LA-105 |  |  |  |
| Corynebacterium glutamicum K-38 |  | 7087 |  |
| Corynebacterium glutamicum K-43 |  | 7162 |  |
| Corynebacterium herculis |  |  | 13868 |
| Corynebacterium herculis L-103 |  | 5947 | 31866 |
| Corynebacterium acetoacidophilum |  |  | 13870 |
| Corynebacterium lilium |  |  | 15990 |
| Brevibacterium divaricatum |  |  | 14020 |
| Brevibacterium divaricatum L-204 |  | 5948 | 31867 |
| Brevibacterium lactofermentum |  |  | 13869 |
| Brevibacterium lactofermentum L-312 |  | 5949 | 31868 |
| Brevibacterium flavum |  |  | 14067 |
| Brevibacterium immariochilium |  |  | 14068 |
| Brevibactrerium thiogenitalis |  |  | 19240 |

Transformation of the host microorganisms with recombinant DNAs is carried out by the following steps:

1) Preparation of protoplasts from cultured cells;

2) Transformation of the protoplasts with a recombinant DNA;

3) Regeneration of the protoplasts to normal cells and selection of a transformant;

These steps are described in detail below.

1. Preparation of protoplasts from cultured cells:

The preparation of protoplasts is carried out by culturing a microorganism under conditions which render it sensitive to lysozyme, a lytic enzyme, and treating the cultured cells with lysozyme in a hypertonic solution to remove the cell wall. In order to render microbial cells sensitive to lysozyme, reagents inhibiting the synthesis of bacterial cell walls are used. For example, microbial cells sensitive to lysozyme are obtained by adding, during the logarithmic growth phase, an amount of penicillin which does not inhibit or sub-inhibits the growth and then continuing culturing for several generations.

For culturing, any medium wherein the microorganism can grow may be used. For example, a nutrient medium NB (pH 7.2) consisting of 20 g/ℓ powdered bouillon and 5 g/ℓ yeast extract and a semi-synthetic medium SSM (pH 7.2) consisting of 10 g/ℓ glucose, 4 g/ℓ $NH_4Cl$, 2 g/ℓ urea, 1 g/ℓ yeast extract, 1 g/ℓ $KH_2PO_4$, 3 g/ℓ $K_2HPO_4$, 0.4 g/ℓ $MgCl_2 \cdot 6H_2O$, 10 mg/ℓ $FeSO_4 \cdot 7H_2O$, 0.2 mg/ℓ $MnSO_4 \cdot (4-6)H_2O$, 0.9 mg/ℓ $ZnSO_4 \cdot 7H_2O$, 0.4 mg/ℓ $CuSO_4 \cdot 5H_2O$, 0.09 mg/ℓ $Na_2B_4O_7 \cdot 10H_2O$, 0.04 mg/ℓ $(NH_4)_6MO_7O_{24} \cdot 4H_2O$, 30 μg/ℓ biotin and 1 mg/ℓ thiamine hydrochloride are used.

Microbial cells are inoculated in the medium and culturing is carried out with shaking.

The optical density (OD) of the culture medium at 660 nm is monitored with a colorimeter and penicillin, such as penicillin G, is added to the medium at an initial stage of the logarithmic growth phase (OD : 0.1 - 0.4) in a concentration of 0.1 to 2.0 U/mℓ. Culturing is continued to an OD value of 0.3 - 0.5, and then cells are harvested and washed with the SSM medium. The washed cells are resuspended in a suitable hypertonic medium such as PFM medium (pH 7.0 - 8.5) wherein 0.4M sucrose and 0.01M $MgCl_2 \cdot 6H_2O$ are added to 2 fold diluted SSM medium, and RCG medium (pH 7.0 - 8.5) consisting of 5 g/ℓ glucose, 5 g/ℓ casein hydrolysate, 2.5 g/ℓ yeast extract, 3.5 g/ℓ $K_2HPO_4$, 1.5 g/ℓ $KH_2PO_4$, 0.41 g/ℓ $MgCl_2 \cdot 6H_2O$, 10 mg/ℓ $FeSO_4 \cdot 7H_2O$, 2 mg/ℓ $MnSO_4 \cdot (4-6)H_2O$, 0.9 mg/ℓ $ZnSO_4 \cdot 7H_2O$, 0.4 mg/ℓ $CuSO_4 \cdot 5H_2O$, 0.09 mg/ℓ $Na_2B_4O_7 \cdot 10H_2O$, 0.04 mg/ℓ $(NH_4)_6Mo_7O_{24} \cdot 4H_2O$, 30 μg/ℓ biotin, 2 mg/ℓ thiamine hydrochloride and 135 g/ℓ sodium succinate or RCGP medium which consists of RCG medium and 3% polyvinyl pyrrolidone. To the cell suspension, lysozyme is added to a final concentration of 0.2 to 10 mg/mℓ, and the mixture is allowed to react at a temperature of 30 to 37 °C. Protoplast formation proceeds with time and is monitored with an optical microscope. The period required for the conversion of most cells to protoplasts depends on the concentrations of the penicillin used for the lysozyme-sensitization and the amount of lysozyme used. The period is 3 - 24 hours under the conditions mentioned above.

5

Since protoplasts formed are destroyed under hypotonic conditions, the extent of the formation of protoplast is determined indirectly from the number of normal cells surviving under hypotonic conditions. Generally, the ratio of surviving normal cells are kept below $10^{-4}$ per lysozyme-treated normal cell.

The protoplasts as prepared above have colony-forming (regenerating) ability on a suitable hypertonic agar medium. As the agar medium, a nutrient medium, a semi-synthetic medium or a synthetic medium containing various amino acids, which contains 0.3 to 0.8M sodium succinate and 0.5 to 6% polyvinyl pyrrolidone with a molecular weight of 10,000 or 40,000 is preferably used. Generally, a semi-synthetic medium RCGP (pH 7.2) wherein 1.4% agar is added to the RCGP agar medium is used. Regeneration is carried out at a temperature of 25 to 35°C. The cultivation time required for the regeneration of protoplasts depends upon the strain used, and usually in 10 to 14 days colonies can be picked up. The efficiency of the reneration of protoplasts on the RCGP medium also depends on the strain used, the concentrations of the penicillin added during the cultivation and the concentration of lysozyme used. The efficiency is generally $10^{-2}$ - $10^{-4}$ cells per normal cell treated with lysozyme.

## 2. Transformation of the protoplasts with a recombinant DNA:

Introduction of recombinant DNA into the protoplast is carried out by mixing the protoplast and the DNA in a hypertonic solution which protects the protoplast and by adding to the mixture polyethyleneglycol (PEG, average molecular weight: 1,540 - 6,000) or polyvinylalcohol (PVA, degree of polymerization: 500 - 1,500) and a divalent metal cation which stimulates the uptake of DNA. As a stabilizing agent for the hypertonic conditions, those generally used to protect protoplasts of other microorganisms such as sucrose and sodium succinate are also employed. PEG and PVA can be used at a final concentration of 5 to 60% and 1 to 20%, respectively. Divalent metal cations such as $Ca^{++}$, $Mg^{++}$, $Mn^{++}$, $Ba^{++}$ and $Sr^{++}$ are effectively used alone or in combination at a final concentration of 1 to 100 mM. Transformation is carried out satisfactorily at 0 to 25°C.

## 3. Regeneration of the protoplasts to normal cells and selection of a transformant:

Regeneration of the protoplast transformed with a recombinant DNA is carried out in the same way as mentioned above by spreading the protoplast on a hypertonic agar medium such as RCGP medium containing sodium succinate and polyvinyl pyrrolidone and incubating at a temperature wherein normal cells can grow, generally 25 to 35°C. Transformants are obtained by selecting the phenotypes derived from donor DNAs. The selection by characteristic phenotype endowed may be carried out simultaneously with regeneration on a hypertonic agar medium or may be carried out on a hypotonic agar medium after non-selective reversion to normal cells on a hypertonic agar medium.

In the case of using the lysozyme-sensitive strains described as the preferred host microorganisms, transformation may be carried out by the steps described in (1) to (3) except that the cultured cells are directly treated with lysozyme without prior treatment with penicillin in step (1). In that case, transformants are obtained at an efficiency of $10^{-2}$ to $10^{-4}$ per regenerated cell.

The following strains are the examples of the transformants obtained by the present invention.

Tyrosine-producing strains
Corynebacterium glutamicum K44, FERM P-7163
Corynebacterium glutamicum K45, FERM P-7164

The phenotypic expression of the recombinant DNA is carried out by growing the transformant in a conventional nutrient medium. Appropriate reagents may be added to the medium according to the phenotypes expected from the gene DNA or vector DNA on the recombinant DNA.

The thus obtained transformant is cultured in a conventional manner used in the production of amino acids by fermentation. That is, the transformant is cultured in a conventional medium containing carbon sources, nitrogen sources, inorganic materials, amino acids, vitamines, etc. under aerobic conditions, with adjustment of temperature and pH. Amino acids, thus accumulated in the medium are recovered.

As the carbon source, various carbohydrates such as glucose, glycerol, fructose, sucrose, maltose, mannose, starch, starch hydrolyzate and molasses, polyalcohols and various organic acids such as pyruvic acid, fumaric acid, lactic acid and acetic acid may be used. According to the assimilability of the microorganism strain used, hydrocarbon and alcohols are employed. Blackstrap molasses is most preferably used.

As the nitrogen source, ammonia, various inorganic or organic ammonium salts such as ammonium chloride, ammonium sulfate, ammonium carbonate and ammonium acetate, urea, and nitrogenous organic substances such as peptone, NZ-amine, meat extract, yeast extract, corn steep liquor, casein hydrolyzate, fish meal or its digested product, defatted soybean or its digested product and chrysalis hydrolyzate are appropriate.

As the inorganic materials, potassium dihydrogenphosphate, dipotassium hydrogenphosphate, ammonium sulfate, ammonium chloride, magnesium sulfate, sodium chloride, ferrous sulfate, manganese sulfate and calcium carbonate may be used. Vitamines and amino acids required for the growth of microorganisms may not be added, provided that they are supplied with other components mentioned above.

Culturing is carried out under aerobic conditions with shaking or aeration-agitation. Culturing temperature is preferably 20 to 40°C. The pH of the medium during culturing is maintained around neutral. Culturing is continued until a considerable amount of an amino acid is accumulated, generally for 1 to 5 days.

After completion of the culturing, cells are removed and the amino acid is recovered from the culture liquor by conventional manners such as treatment with active carbon or ion exchange resin.

In spite of the high similarity in microbiological characteristics, so called glutamic acid-producing microorganisms which produce glutamic acid in large amounts are classified into various species and even into different genera such as Corynebacterium and Brevibacterium, which is probably because of their industrial importance. However, it has been pointed out that these microorganisms should belong to one species because of nearly the same composition of amino acids in the cell wall and the base composition of DNAs. Recently, it has been reported that these microorganisms have at least 70 to 80% homology in DNA-DNA hybridization, indicating that these microorganisms are closely related. See, e.g., Komatsu, Y.: Report of the Fermentation Research Institute, No. 55, 1 (1980), and Suzuki, K., Kaneko, T., and Komagata, K.: Int. J. yst. Bacteriol., 31, 131 (1981).

In consideration of the facts mentioned above, it is readily assumed that the usefulness of the present invention is applicable to all the glutamic acid-producing microorganisms. In order to stably maintain recombinant DNA molecules and express the DNA in these species, slight differences of such properties of the host microorganisms as homology in the DNA are negligible and it is sufficient for host microorganisms to allow the autonomous replication of plasmids and expression of genes on them. That these microorganisms have such abilities is apparent from the facts that plasmid pCG4 which was isolated from Corynebacterium glutamicum 225-250 (Japanese Published Unexamined Patent Application No. 183799/82) and having an streptomycin and/or spectinomycin resistant gene could replicate in the glutamic acid-producing microorganisms such as those belonging to the genus Corynebacterium or Brevibacterium and that the gene responsible for the resistance could be expressed (Japanese Published Unexamined Patent Application No. 186492/82). Further, as described in the production of histidine of the parent application of this application, which matured into European Patent No. 136 359, it is apparent that the gene expressed in Corynebacterium glutamicum is expressible in broad host microorganisms of the genera Corynebacterium, Brevibacterium and the like. Therefore, all the glutamic acid-producing microorganisms including those belonging to the genus Corynebacterium or Brevibacterium as well as the species Corynebacterium glutamicum are competent as the host microorganism of the present invention.

For the strains appearing in the present specification, the accession numbers, deposition date and transfer date of the deposits under the Budapest Treaty of the International Recognition of the Deposit of Microorganisms for the purpose of Patent Procedure are as follows.

| | ATCC | FERM P (BP) | Deposition date (Transfer date) Year/Month/Day |
|---|---|---|---|
| Corynebacterium glutamicum L-15 | 31834 | | 1981. 3. 9 ( ) |
| Corynebacterium herculis L-103 | 31366 | | 1981. 4. 3 ( ) |
| Brevibacterium divaricatum L-204 | 31367 | | 1981. 4. 3 ( ) |
| Brevibacterium lactofermentum L-312 | 31368 | | 1981. 4. 3 ( ) |
| Corynebacterium glutamicum LA103/pCES4 | 39019 | | 1981.12.11 |
| Corynebacterium glutamicum LA103/pCB101 | 39020 | | 1981.12.11 |
| Corynebacterium glutamicum LA103/pEthr1 | 39021 | | 1981.12.11 |
| Corynebacterium glutamicum LA103/pCG11 | 39022 | | 1981.12.11 |
| Corynebacterium glutamicum K17 | 39032 | | 1981.12.21 |
| Corynebacterium glutamicum K18 | 39033 | | 1981.12.21 |
| Corynebacterium glutamicum K19 | 39034 | | 1981.12.21 |
| Corynebacterium glutamicum K20 | 39035 | | 1981.12.21 |
| Corynebacterium glutamicum K31 | 39280 | | 1983. 1.28 |
| Corynebacterium glutamicum K32 | 39281 | | 1983. 1.28 |
| Corynebacterium herculis K33 | 39282 | | 1983. 1.28 |
| Brevibacterium fluvum K34 | 39283 | | 1983. 1.28 |
| Brevibacterium lactofermentum K35 | 39284 | | 1983. 1.28 |

| | | |
|---|---|---|
| Corynebacterium glutamicum K37 | 39285 | 1983. 1.31 |
| Corynebacterium glutamicum K36 | 6908 ( 451) | 1983. 2. 3 (1984. 1.19) |
| Corynebacterium glutamicum H23 | 6909 ( 452) | 1983. 2. 3 (1984. 1.19) |
| Corynebacterium glutamicum C156 | 6910 ( 453) | 1983. 2. 8 (1984. 1.19) |
| Corynebacterium glutamicum K38 | 7087 ( 454) | 1983. 5.19 (1984. 1.19) |
| Corynebacterium glutamicum K39 | 7088 ( 459) | 1983. 5.19 (1984. 1.21) |
| Corynebacterium glutamicum K40 | 7160 ( 455) | 1983. 7.21 (1984. 1.19) |
| Corynebacterium glutamicum K41 | 7161 ( 456) | 1983. 7.21 (1984. 1.19) |
| Corynebacterium glutamicum K43 | 7162 ( 457) | 1983. 7.21 (1984. 1.19) |
| Corynebacterium glutamicum K44 | 7163 ( 458) | 1983. 7.21 (1984. 1.19) |
| Corynebacterium glutamicum K45 | 7164 ( 460) | 1983. 7.21 (1984. 1.21) |
| Brevibacterium fluvum K42 | ( 355) | 1983. 9.12 |
| Corynebacterium glutamicum K46 | ( 356) | 1983. 9.12 |
| Brevibacterium fluvum K48 | ( 357) | 1983. 9.12 |
| Corynebacterium herculis K47 | ( 367) | 1983. 9.21 |
| Corynebacterium glutamicum K49 | ( 464) | 1984. 1.25 |

Fig. 1 illustrates the cleavage pattern with restriction endonucleases for plasmid pEaroF-1.
Fig. 2 illustrates the cleavage pattern with restriction endonucleases for plasmid pKmlaroFl.

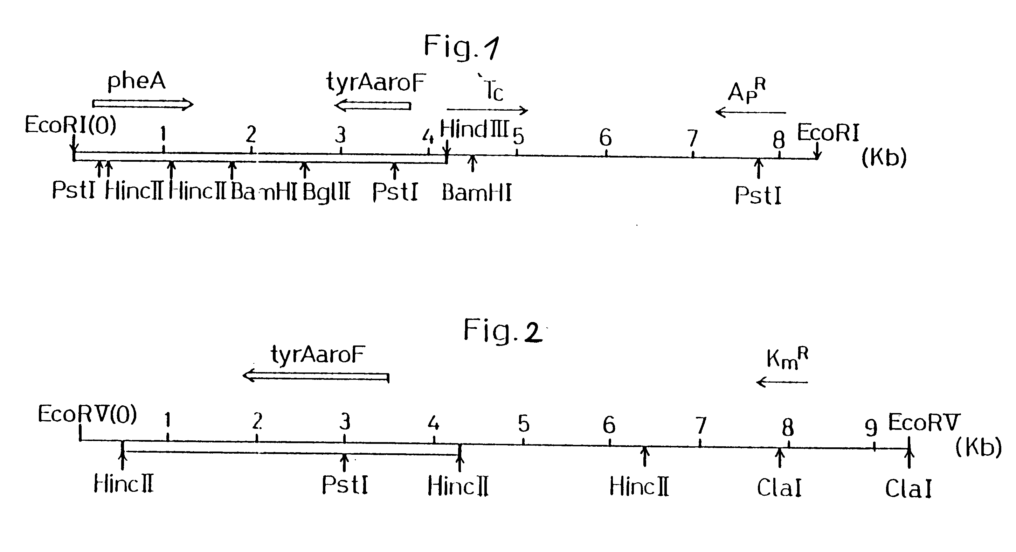

In Figs. 1 and 2 the horizontal arrows show orientation of transcription of genes.

EP 0 332 234 B1

Example 1

Production of tyrosine:

(1) Preparation of the chromosomal DNA and plasmid DNA:

The chromosomal DNA of Escherichia coli JA194 [Proc. Natl. Acad. Sci., 94, 487-491 (1977)] was prepared by the following method.

A seed culture was inoculated into 400 mℓ of LB (pH 7.0, the same LB was used hereinafter). Culturing was carried out with shaking at 37°C and continued to a latter stage of the logarithmic growth phase. Cells were harvested from the culture broth and high molecular chromosomal DNAs were isolated from the cells by the method of Saito et al.

Separately pBR322 used as a vector was prepared from the cultured cells of Escherichia coli JA194 harboring pBR322 by the following method.

The strain was grown with shaking at 37°C in 400 mℓ of LB containing 100 $\mu$g/mℓ ampicillin to a latter stage of the logarithmic growth phase. Cells were harvested from the culture broth and lysed by the method of Tanaka, et al., [J. Bacteriol. 121, 354-362 (1975)].

The whole lysate was centrifuged at 4°C at 28,000 rpm for one hour. The supernatant fluid was recovered and one fifth volume of 50% (W/V) polyethyleneglycol (PEG) 6,000 aqueous solution was added. The mixture was stirred slowly and allowed to stand at 4°C overnight. Formed precipitates were collected by centrifugation at 3,000 rpm at 4°C for 5 minutes and dissolved in 5 mℓ of TE buffer solution (pH 7.5) consisting of 10 mM Tris and 1 mM EDTA•$Na_2$ (the same TE buffer solution was used hereinafter). Then, one mℓ of 1.5 mg/mℓ ethidium bromide was added, and TE buffer solution was added to the total volume of 7.5 mℓ. To the mixture was added 7.875 g of CsCl, and dissolved completely. The solution was centrifuged at 105,000 x g at 20°C for 40 hours. A plasmid band detected under UV irradiation was taken out with an injector. Ethidium bromide was extracted three times with isopropanol containing 15% (V/V) TE buffer solution. The residue was dialysed against TE buffer solution at 4°C overnight, and used as a plasmid DNA.

(2) Cloning of a DNA fragment containing the gene coding for DAHPase, CMase and PDGase:

In this step, 5 units each of restriction enzymes EcoRI and HindIII were added to 100 $\mu$ℓ of a HindIII reaction solution containing 3 $\mu$g of pBR322 plasmid DNA prepared above, and 5 units each of EcoRI and HindIII was added to 100 $\mu$ℓ of restriction enzyme HindIII reaction solution containing 9 $\mu$g of the chromosomal DNA. Each of the mixtures was allowed to react at 37°C for 60 minutes and heated at 65°C for 10 minutes to stop the reaction. Both of the mixtures were admixed with each other. Then, 40 $\mu$ℓ of a T4 ligase buffer solution II, 40 $\mu$ℓ of 5 mM ATP, 0.4 $\mu$ℓ of T4 ligase and 120 $\mu$ℓ of $H_2O$ were added. The mixture was allowed to react at 12°C for 16 hours.

The above ligation mixture was provided for the following transformation. As the recipient for the transformation, DAHPase-deficient Escherichia coli AB3248 strain [J. Bact., 93, 237-244 (1967)] or tyrA gene (CMase and PDGase)-deficient Escherichia coli AT2273 strain [J. Bact., 91, 1494 (1966)] was used. The seed culture of the strain was inoculated into LB and competent cells were prepared according to the method of M. Dagert, (Gene, 6, 23-28 (1979)].

Fifty microliter of the ligation mixture was added to 0.2 mℓ of a solution containing $10^9$/mℓ competent cells and the mixture was allowed to stand under ice cooling for 10 minutes. After heating at 37°C for 5 minutes, 2 mℓ of LB was added, followed by standing at 37°C for 90 to 120 minutes. Cells were subjected to washing with physiological saline solution and centrifugation twice. The cells were spread on M9 plate medium (pH 7.0) consisting of 1 g/ℓ $NH_4Cl$, 6 g/ℓ $Na_2HPO_4$, 3 g/ℓ $KH_2PO_4$, 5 g/ℓ NaCl, 0.1 g/ℓ $MgSO_4$•$7H_2O$, 0.015 g/ℓ $CaCl_2$•$2H_2O$, 3 g/ℓ glucose, 4 mg/ℓ vitamine B1 and 15 g/ℓ agar, [J. Bact. 121, 354-362 (1975), the same M9 plate medium was used hereinafter] and containing 50 $\mu$g/mℓ each histidine, proline, arginine, isoleucine and valine. Colonies grown on M9 plate medium were plated on LB plate media containing 100 $\mu$g/mℓ ampicillin and 20 $\mu$g/mℓ tetracycline, respectively. Colonies which grew on the ampicillin-containing medium and did not grow on the tetracycline-containing medium were selected. Plasmid DNAs were isolated from the thus selected transformants which grew on M9 plate medium containing histidine, proline, arginine, isoleucine and valine and which were resistant to ampicillin and sensitive to tetracycline by the same method as described above. Plasmid pEaroFl isolated from one of the transformants was digested with various restriction endonucleases and analysed by agarose gel electrophoresis. The analysis showed that an EcoRI-HindIII cleaved DNA fragment of about 4.2 Kb was inserted into the larger EcoRI-HindIII cleaved fragment of pBR322.

Escherichia coli. AB3248 and Escherichia coli AT2273 were transformed by the same method as described above using pEaroFl obtained above. Both transformants grew on M9 medium containing histidine, proline, arginine, isolecuine and valine and simultaneously were resistant to ampicillin. The fact showed that the transformants had the same plasmids as pEaroFl.

The results described above showed that the genes encoding for DAHPase, CMase and PDGase exist on the DNA fragment of about 4.2 Kb cloned in pEaroFl.

Further, pEaroFl having cleavage sites with various restriction endonucleases such as EcoRI, BamHI, HindIII and the like as shown in Fig. 1 was compared with plasmid pKB45 reported by G. Zurawski, Proc. Natl. Acad. Sci. USA $\underline{75}$, 4271 (1978) to show that the DNA fragment of about 4.2 Kb in pEaroFl possesses aroF, tyrA and pheA genes of Escherichia coli.

(3) Subcloning of aroFtyrA gene:

A DNA fragment containing aroFtyrA gene was recovered from plasmid pEaroFl DNA prepared above and ligated to pCE51 which is a shuttle vector for Escherichia coli and Corynebacterium glutamicum.

pCE51 is a recombinant plasmid wherein plasmid pCG1 (Japanese Published Unexamined Patent Application No. 134500/82) of Corynebacterium glutamicum is ligated with plasmid pGA22 of Escherichia coli [refer to An, G. et al: J. Bacteriol., $\underline{140}$, 400 (1979)]. The ligation is carried out using the same cohesive ends of BglII cleaved pCG1 and BamHI fragment of pGA22 containing the kanamycin-resistant gene.

pCE51 is practically prepared by the method described in Japanese Published Unexamined Patent Application Nos. 105999/83 and 126789/83.

Five units of HincII was added to 100 $\mu$l of HincII reaction solution consisting of 10 mM Tris-HCl (pH 8.0), 7 mM MgCl$_2$ and 60 mM NaCl and containing 3 $\mu$g of plasmid pEaroFl DNA and reaction was carried out at 37°C for 60 minutes. Three-tenth units of HincII was added to 100 $\mu$l of HincII reaction solution containing 3 $\mu$g of plasmid pCE51 DNA prepared from pCE51-carrying Escherichia coli JA194 strain by the same method as mentioned above and reaction was carried out at 37°C for 60 minutes to cut pCE51 at one of two HincII cleavage sites. Both of the reaction mixtures were mixed with each other. 40 $\mu$l of T4 ligase buffer solution II, 40 $\mu$l of 5 mM ATP, 0.4 $\mu$l of T4 ligase and 120 $\mu$l of water were added. The mixture was allowed to react at 12°C for 16 hours and reaction was stopped by heating at 65°C for 10 minutes.

The ligation mixture was provided for the following transformation. As the recipient for the transformation, Escherichia coli AB3248 strain was used. Transformation was carried out by the same method as described above to obtain colonies grown on M9 plate medium containing histidine, proline, arginine, isoleucine and valine. Colonies grown on LB plate medium containing 20 $\mu$g/ml kanamycin were selected from the colonies obtained above.

Plasmid DNAs were isolated from the transformants which grew on M9 plate medium containing histidine, proline, arginine, isoleucine and valine and were resistant to kanamycin by the same method as described above. Plasmid pKmIaroFl obtained from one of the transformants were digested with various restriction endonucleases and analysed by agarose gel electrophoresis. The analysis showed that a HincII cleaved DNA fragment of about 3.8 Kb bearing aroFtyrA of pEaroFl was inserted in one of two HincII cleavage sites of pCE51.

Plasmid pKmIaroFl obtained above has the restriction pattern as illustrated in Fig. 2.

(4) Preparation of tyrosine- and tyrosine analog-resistant plasmid pKmIaroFl-m-18 from pKmIaroFl-carrying strain:

pKmIaroFl-carrying AB3248 strain was grown in LB medium containing 20 $\mu$g/ml kanamycin to a latter stage of the logarithmic growth phase. Cells were harvested by centrifugation and washed with 50 mM Tris-malate buffer solution (pH 6.0) twice and incubated with 400 $\mu$g/ml N-methyl-N'-nitro-N-nitrosoguanidine in 50 mM Tris-malate buffer solution (pH 6.0) at room temperature for 30 minutes. The treated cells were harvested by centrifugation and washed with 50 mM Tris-malate buffer solution (pH 6.0) twice. The washed cells were cultured in LB medium containing 20 $\mu$g/ml kanamycin at 30°C for 16 hours and plasmid DNAs were isolated by the same method as mentioned above.

Escherichia coli AB3248 was transformed using the isolated plasmids. Selection of transformants was carried out on M9 plate medium containing 0.25 mg/ml tyrosine and 50 $\mu$g/ml each histidine, proline, arginine, isoleucine and valine. Colonies grown on M9 plate medium containing 0.25 mg/ml tyrosine and 50 $\mu$g/ml each histidine, proline, arginine, isoleucine and valine and on LB plate medium containing 20 $\mu$g/ml

kanamycin were selected from the developed colonies.

The thus obtained colonies were resistant to 3-aminotyrosine (3AT), a tyrosine analog (product of Shigma Co.) since they could grow on M9 plate medium containing 0.2 mg/ml 3AT and 50 $\mu$g/ml each histidine, proline, arginine, isoleucine and valine.

The plasmids obtained from the thus prepared transformant can confer resistance to tyrosine or a tyrosine analog on a microorganism. Escherichia coli AB3248 strain carrying one of such plasmids, pKmIaroF-m-18 was able to grow on M9 plate medium containing 1 mg/ml tyrosine or 0.5 mg/ml 3AT, and 50 $\mu$g/ml each histidine, proline, arginine, isoleucine and valine.

(5) Transformation of Corynebacterium glutamicum K43 with pKmIaroFl and pKmIaroFl-m-18:

A seed culture of Corynebacterium glutamicum K43 (FERM P-7162, FERM BP-457) was inoculated in Semi-synthetic medium SSM containing 50 $\mu$g/ml phenylalanine and culturing was carried out with shaking at 30°C. NB medium was used for seed culture. The optical density (OD) at 660 nm was monitored with a Tokyo Koden colorimeter and, at an OD value of 0.2, penicillin G was added to the broth to a final concentration of 0.5 unit/ml. Culturing was continued to an OD value of about 0.6.

Cells were harvested at an OD value of 0.6. The cells were suspended at about $10^9$ cells/ml in RCGP medium containing 1 mg/ml lysozyme. The suspension was put in an L-tube and stirred slowly at 30°C for 5 hours to obtain protoplasts. Then, 0.5 ml of the protoplast suspension was put in a small test tube and centrifuged at 2,500 x g for 5 minutes. The protoplasts were resuspended in 1 ml of TSMC buffer and again subjected to centrifugation and washing. The washed protoplasts were resuspended in 0.1 ml of TSMC buffer solution. One hundred microliter of a mixture (1 : 1 by volume) of a two-fold concentrated TSMC buffer and the ligated DNA mixture described above was added to the protoplast suspension. Then, 0.8 ml of a solution containing 20% PEG 6,000 in TSMC buffer solution was added to the mixture. Plasmid DNAs, pKmIaroFl and pKmIarcFl-m-18 were prepared from Escherichia coli AB3248 carrying these plasmids as described above. After 3 minutes, 2 ml of RCGP medium (pH 7.2) was added and the mixture was centrifuged at 2,500 x g for 5 minutes. The supernatant fluid was removed and the precipitated protoplasts were suspended in 1 ml of RCGP medium. Then, 0.2 ml of the suspension was spread on RCGP agar medium containing 200 $\mu$g/ml kanamycin and incubated at 30°C for 7 days. Colonies resistant to kanamycin were grown on the selection plate.

(6) Production of tyrosine by the transformant:

The thus obtained transformants carrying pKmIaroFl and pKmIaroFl-m-18 have been deposited with the Fermentation Research Institute as Corynebacterium glutamicum K44 (FERM P-7163, FERM BP-458) and Corynebacterium glutamicum K45 (FERM P-7164, FERM BP-460), respectively.

L-tyrosine production by pKmIaroFl and pKmIaroFl-m-18-carrying strains was carried out as follows.

The strain was cultured in NB aqueous medium at 30°C for 16 hours and 0.5 ml of the culture broth was inoculated in 5 ml of a production medium P4 (pH 7.2) consisting of 100g/l molasses, 20g/l $(NH_4)_2SO_4$, 0,5g/l $KH_2PO_4$, 0.5g/l $K_2HPO_4$, 0.25g/l $MgSO_4 \cdot 7H_2O$ and 20g/l $CaCO_3$ containing 0.25% NZ amine. Culturing was carried out at 30°C for 96 hours.

After culturing, 6N NaOH solution was added to the broth to a concentration of 50 $\mu$l/ml and the mixture was heated at 65°C for 5 minutes to dissolve precipitated tyrosine completely. The culture filtrate was subjected to paper chromatography and color reaction with ninhydrin, and the amount of L-tyrosine formed was determined colorimetrically. As a control, Corynebacterium glutamicum K43 was similarly treated. The results are shown in Table I.

Table I

| Strain | Amount of L-tyrosine (mg/ml) |
|---|---|
| Corynebacterium glutamicum K43 | 4.8 |
| Corynebacterium glutamicum K44 | 5.3 |
| Corynebacterium glutamicum K45 | 7.7 |

12

## Claims

1. A process for producing L-tyrosine which comprises culturing in a medium a microorganism obtainable by transforming a host microorganism belonging to the genus Corynebacterium or Brevibacterium with a recombinant DNA wherein a DNA fragment containing a gene coding for 3-deoxy-2-keto-D-arabino-heptulosonate-7-phosphate synthetase, chorismate mutase and prephenate dehydrogenase is inserted into a vector DNA, accumulating L-tyrosine in the culture and recovering L-tyrosine therefrom.

2. The process according to claim 1, wherein the recombinant DNA is plasmid pkmlaroFl (FERM BP-458) or plasmid pkmlaroFl-m-18 (FERM BP-460).

3. The process according to claim 1, wherein the microorganism is Corynebacterium glutamicum K44, FERM BP-458 or Corynebacterium glutamicum K45, FERM BP-460.

4. A biologically pure culture of Corynebacterium glutamicum K44, FERM BP-458 or Corynebacterium glutamicum K45, FERM-BP-460.

## Patentansprüche

1. Verfahren zur Herstellung von L-Tyrosin, bei dem man in einem Medium einen Mikroorganismus züchtet, der durch das Transformieren eines zu der Gattung Corynebacterium oder Brevibacterium gehörenden Wirtsmikroorganismus mit einer rekombinanten DNA erhältlich ist, wobei ein DNA-Fragment, das ein 3-Desoxy-2-keto-D-arabino-heptulosonat-7-phosphat-Synthase,Chorismat-Mutase und Prephenat-Dehydrogenase codierendes Gen enthält, in eine Vektor-DNA inseriert wird, L-Tyrosin in der Kultur ansammelt und L-Tyrosin daraus gewinnt.

2. Verfahren nach Anspruch 1, wobei die rekombinante DNA das Plasmid pkmlaroFl (FERM BP-458) oder das Plasmid pkmlaroFl-m-18 (FERM BP-460) ist.

3. Verfahren nach Anspruch 1, wobei der Mikroorganismus Corynebacterium glutamicum K44, FERM BP-458 oder Corynebacterium glutamicum K45 FERM BP-460 ist.

4. Biologisch reine Kultur von Corynebacterium glutamicum K44, FERM BP-458 oder Corynebacterium glutamicum K45, FERM BP-460.

## Revendications

1. Procédé de production de L-tyrosine, qui comprend le fait de cultiver, dans un milieu, un microorganisme que l'on peut obtenir en transformant un microorganisme hôte, appartenant au genre Corynebacterium ou Brevibacterium, avec un ADN recombinant dans lequel est inséré, au sein d'un ADN vecteur, un fragment d'ADN contenant un gène codant la 3-désoxy-2-céto-D-arabino-heptulosonate-7-phosphatesynthétase, la chorismate mutase et la préphénate déshydrogénase, le fait de laisser la L-tyrosine s'accumuler dans la culture, et le fait de récupérer la L-tyrosine à partir de celle-ci.

2. Procédé conforme à la revendication 1, dans lequel l'ADN recombinant est le plasmide pkmlaroF1 (FERM BP-458) ou le plasmide pkmlaroF1-m-18(FERM BP-460).

3. Procédé conforme à la revendication 1, dans lequel le microorganisme est Corynebacterium glutamicum K44, FERM BP-458, ou Corynebacterium glutamicum K45, FERM BP-460.

4. Culture biologiquement pure de Corynebacterium glutamicum K44, FERM BP-458, ou de Corynebacterium glutamicum K45, FERM BP-460.

Fig.1

Fig.2

EP 0 332 234 B1